# EUROPEAN PATENT APPLICATION

(11) **EP 1 491 195 A1**
(43) Date of publication of application: **29.12.2004**
(21) Application number: 04076764.2
(22) Date of filing: 15.06.2004
(51) Int. Cl.: A61K 31/43, A61K 31/424, A61P 31/04

(54) **PHARMACEUTICAL FORMULATIONS COMPRISING AMOXICILLIN AND CLAVULANATE**

(30) Priority: 16.06.2003 US 478769 P; 16.06.2003 GB 3139136
(71) Applicant: GLAXO GROUP LIMITED, Greenford, Middlesex UB6 0NN (GB)
(72) Inventor: Ramsey, Michael Gale, Glaxosmithkline, Bristol, Tennessee 37620 (US); Rickman, Tracy, Glaxosmithkline, Bristol, Tennessee 37620 (US)
(74) Representative: Connell, Anthony Christopher

(57) **Abstract**

Formulations comprising amoxicillin and clavulanate for reconstitution into an aqueous suspension incorporate low levels of carboxymethylcellulose sodium to stabilise the pH thereof.

## Description

This invention relates to pharmaceutical formulations comprising amoxicillin and clavulanate adapted for paediatric administration.

The combination of amoxicillin and clavulanate (co-amoxiclav) is an effective empirical treatment for bacterial infections and may be administered by oral dosing, for instance in the form of tablets, and, for paediatric formulations, as an aqueous solution or suspension, which is normally flavoured. Various co-amoxiclav formulations are marketed by GIaxoSmithKline under the trade mark Augmentin.

Paediatric formulations comprising amoxicillin and clavulanate are typically provided as a dry powder or granules, in a container such as a bottle. This is reconstituted with a defined volume of water prior to first use, to provide a suspension of defined concentration, generally a multiple dose suspension which covers the entire course of therapy.

Clavulanate is well known to be vulnerable to degradation, both as a dry powder and also in the reconstituted state. Stability in the dry state is important as it can limit the shelf-life of a product. Stability, when constituted as a liquid suspension, is also important as it is necessary to provide product in a liquid state that remains stable over a period of 7 or 10 days when stored in appropriate conditions, for instance at about 5°C (in a refrigerator).

It is usual to include an overage of the clavulanate component so that it retains at least 90% of the nominal amount after 7 or 10 days of storage, thereby retaining optimum efficacy. Even with such overages, it is necessary to carefully control product pH to minimise the rate of clavulanate degradation in the liquid state.

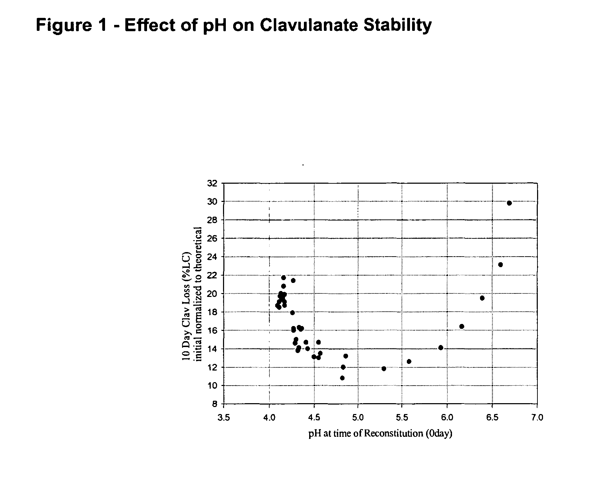

Clavulanate in aqueous solution has a "U-shaped" pH-stability profile, with optimum stability in the region pH 5.0 to 5.5. It is however difficult to keep solutions at optimum pH because degradation causes the pH to increase, due to the formation of basic degradation products. If the pH rises too far, degradation is accelerated, even within the period of 7 or 10 days (as shown in Figure 1).

Maintenance of good stability of the clavulanate component in the liquid state by pH control is complicated by the fact that amoxicillin generates acidic degradation products in aqueous solution. In addition, both amoxicillin and clavulanate contain low levels of degradation products when initially prepared, arising from the fermentation process by which they are prepared. These can increase during storage, manufacture of product, and storage of product as a dry powder prior to reconstitution. Such variability can lead to inconsistent product pH and variable stability in the reconstituted state; hence the need for control.

This problem is further compounded in reconstituted suspensions which have higher concentrations of amoxicillin. Amoxicillin tends to depress pH in a concentration dependent manner. As it is becoming increasingly necessary to utilise higher doses of amoxicillin, to cope with increasing bacterial resistance, the levels included in medications have had to be increased. Thus, suspension formulations of amoxicillin / clavulanate, which were intially 125/32.5 mg/5ml and 250/62.5 mg/5ml have increased to 400/57 mg/5ml and 100/12.5 mg/ml and, more recently, to 600/43 mg/5ml with the introduction of the product Augmentin ES 600® in the USA in late 2001. Furthermore, there is the likelihood that formulations comprising 800 mg/5ml and even 1000 mg/5 ml may be required in the future. Such changes are significant in stability terms because the higher inclusion levels of amoxicillin tend to depress pH to a region where product is less stable.

At the same time, the pH of the product increases both during storage as dry powder and whilst in the liquid state, due to increased formation of basic clavulanate degradation products. If the increase is excessive, product will drift into a region of poor clavulanate stability.

Thus, pH fluctuations due to concentrations of the medicaments, variability of input materials or changes when product is in the reconstituted state can lead to inconsistent stability and a non-robust product.

In the light of such constraints, complexities and trends, there is a great need to identify a material which can have a stabilising effect on clavulanate in amoxicillin/clavulanate suspension.

A dry powder formulation comprises a mixture of different excipients, included for various reasons, for instance to assist the manufacturing process, to keep the product dry, to assist formation of a suitable suspension and to enhance aesthetic attributes such as taste and mouth feel. It is necessary to select from within the different classes of generally used excipients those which do not adversely affect stabiiity. Given the pH sensitivity of clavulanate, it might be expected that ingredients to buffer product pH ought be added, to optimise stability. Clavulanate degradation is however known to be accelerated by buffer salts and other ionic species (Haginaka.J; Nakagawa.T; Uno.T: *Chem Pharm Bull* (1981) **29** (11) 3334-3341 Stability of Clavulanic Acid in Aqueous Solutions). Thus, normally useful pH modifiers like citrate salts should be avoided. Other pH modifiers may also be unsuitable, for instance because of salty taste, bitterness, or other unpalatable features that are undesirable in a liquid product which is administered to children. The choice of excipients is thus constrained by such considerations, so formulations comprise as few excipients as possible, to minimise adverse effects on stability in the liquid state.

Formulations of amoxicillin and clavulanate for constitution as liquid oral suspensions have traditionally included a diluent for potassium clavulanate; a desiccant, typically silicon dioxide; a glidant, typically colloidal silicon dioxide; a suspension aid, typically a combination of xanthan gum and hydroxypropylmethyl cellulose; a sweetener, typically an artificial sweetener such as aspartame; and flavourings, typically a combination of fruit or candy-like flavours, for instance, orange, raspberry and golden syrup. Representative formulations are described in WO 96/34605 (SmithKline Beecham), for a 400/57 and 200/28 mg/5ml suspension, and WO 97/09042 (SmithKline Beecham), for a 600/43 mg/5ml suspension and closely reflect existing commercial products. In addition, such formulations have also included a pH modifier such as succinic acid, to modify the pH of the reconstituted suspension so that it remains within the window of stability for clavulanate. Formulations described in WO 00/03695 (Lek) comprise a citric acid / sodium citrate buffering system. In contrast, WO 98/35672 and WO 01/13883 (SmithKline Beecham Laboratoires Pharmaceutiques) describe dry powder formulations for reconstitution into suspensions which are prepared from granulates of amoxicillin and clavulanate in a ratio of 2:1, with the remainder of the amoxicillin provided by granulates of amoxicillin and which formulations do not comprise an identifiable pH modifier.

It has now been surprisingly found that low levels of carboxymethylcellulose sodium have a beneficial effect on the stability of the clavulanate component when suspensions are constituted as liquids.

Carboxymethylcelluose sodium is a pharmaceutical excipient that is widely used for a variety of purposes, for instance, as a coating agent, a disintegrant for tablets and capsules, a tablet binder or, in liquid products, as a stabilising agent, suspending agent, viscosity-increasing agent, or water absorbing agent (Handbook of Pharmaceutical Excipients, third edition, 2000, American Pharmaceutical Association and Pharmaceutical Press, 87). The use of carboxymethylcellulose sodium in a reconstituted suspension of amoxicillin and clavulanate comprising 100/12.5 mg/ml and 400/57 mg/5ml is described in WO 98/35672 and WO 01/13883 (SmithKline Beecham Laboratoires Pharmaceutique). It would however appear to have been included as a conventional suspending or thickening agent, with no recognition of the beneficial effect now observed, due to the relatively lower levels of amoxicillin being used.

Accordingly, the present invention provides for a pharmaceutical formulation of amoxicillin and clavulanate provided as a dry powder composition adapted for constitution with water into a multiple dose suspension which comprises from about 400 to about 1250 mg amoxicillin and from about 40 to about 90 mg clavulanate per unit amount of formulation, such that the ratio of amoxicillin to clavulanate is at least 10:1, and which further comprises a pH stabilising agent which is carboxymethylcelluose sodium. Incorporation of carboxymethylcellulose sodium is found to provide not only optimum pH on initial constitution but also to minimise pH change during storage for ten days, without having any adverse catalytic effect on clavulanate degradation. This effect is surprising in that carboxymethylcellulose sodium is ionic in nature and, as such, might have been expected to catalyse clavulanate degradation. This can be contrasted with the earlier use of the neutral cellulose derivative, hydroxypropylmethyl cellulose, in for instance, the product comprising 600/43 mg/5ml (WO 97/09042) where a similar beneficial pH effect is not seen.

In a further aspect, the present invention provides for a pharmaceutical formulation of amoxicillin and clavulanate provided as a reconstituted suspension which has a concentration in the range about 400 to about 1250 mg amoxicillin and about 40 to about 90 mg clavulanate per 5 ml of reconstituted suspension, such that the ratio of amoxicillin to clavulanate is at least 10:1, and which further comprises as a pH stabilising agent which is carboxymethylcelluose sodium.

In a further aspect, the present invention provides for the use of carboxymethylcellulose sodium in stabilising the pH of a reconstituted suspension comprising from about 400 to about 1250 mg amoxicillin and from about 40 to about 90mg clavulanate per 5 ml of reconstituted suspension, such that the ratio of amoxicillin to clavulanate is at least 10:1, thereby reducing the amount of degradation of clavulanate.

In one embodiment, the pH is in the range about 5.0 to about 5.5 on initial constitution. Preferably, the pH change (increase) during storage is less than about 1 pH unit, preferably less than 0.9 pH units, more preferably less than 0.8 pH units, after 10 days at 4 deg C. Preferably, the final pH, after 10 days storage at 4 deg C is from about 5.8 to about 5.9. This can be contrasted with an initial pH of about 4.1 to 4.6, a subsequent drift in pH of at least 1.5 units and a final pH of from 5.8 to 6.1, for comparable formulations, in the absence of carboxymethylcellulose sodium, and under the same conditions. The present marketed 600/43 mg/5ml product has an initial pH of about 4.7.

In a further aspect,the present invention provides for a pharmaceutical formulation of amoxicillin and clavulanate provided as a dry powder composition adapted for constitution with water into a multiple dose suspension which comprises from about 400 to about 1250 mg amoxicillin and from about 40 to about 90 mg clavulanate per unit amount of formulation, such that the ratio of amoxicillin to clavulanate is at least 10:1, and which on constitution with water has an initial pH of about 5.0 to about 5.5. Preferably, the final pH, after ten days storage at 4 deg C is from about 5.8 to about 5.9.In a further aspect, the present invention provides for the use of carboxymethylcellulose sodium in reducing the amount of degradation of clavulanate in a reconstituted suspension comprising from about 400 to about 1250 mg amoxicillin and from about 40 to about 90 mg clavulanate per unit amount of formulation. Preferably, the loss of clavulante over 10 days storage at 4 deg C is reduced by at least 15%; more preferably, for a reconstituted suspension which has a concentration in the range about 750 to about 1250 mg amoxicillin and about 40 to about 90 mg clavulanate per unit amount of formulation, at least 20%, compared to the same suspension but without carboxymethylcellulose sodium.

In a further aspect of the above mentioned inventions, the suspension comprises from about 500 to about 1250 mg amoxicillin and from about 40 to about 90 mg clavulanate per unit amount of formulation, such that the ratio of amoxicillin to clavulanate is at least 12:1.

In a further aspect of the above mentioned inventions, the suspension comprises from about 550 to about 1250 mg amoxicillin and from about 40 to about 90 mg clavulanate per unit amount of formulation, such that the ratio of amoxicillin to clavulanate is at least 14:1.

In a further aspect of the present invention, amoxicillin is present in the range from about 700 to about 1250 mg per unit amount of formulation and from about 40 to about 90 mg clavulanate per unit amount of formulation, such that the ratio of amoxicillin to clavulanate is at least 14:1.

In a further aspect of the present invention, clavulanate is present in the range from about 40 to about 60 mg per unit amount of formulation, such that the ratio of amoxicillin to clavulanate is at least 14:1.

The ratio of amoxicillin to clavulanate is in the range about 10:1 to 30:1, preferably 12:1 to about 30:1 (with respect to the weights of the corresponding free acids). Typical ratios of amoxicillin and clavulanate include about 14:1, about 15:1 ,about 16:1 and about 20:1, preferably about 14:1 or about 16:1. It will however be appreciated by the skilled man that the critical features are individual absolute amounts of amoxicillin and clavulanate *per se,* rather than the ratio thereof.

Typical compositions comprise about 600, 750, 800, 1000 and 1200 mg amoxicillin per 5 ml of reconstituted suspension, in particular about 600/43 and about 800/57 (ratio 14:1), about 750/50 (ratio 15:1), about 800/50 and about 1000/62.5 (ratio 16:1), and about 800/40 and about 1000/50 (ratio 20:1) mg/5 ml (amoxicillin/clavulanate).

Carboxymethylcellulose sodium is present in an amount effective to provide a pH in the range about 5.0 to about 5.5 on initial constitution and thereafter to minimise pH change during storage, for instance less than about 1 pH unit, preferably less than about 0.9 pH units, more preferably less than about 0.8 pH units, after 10 days storage at 4 deg C. Preferably, the final pH, after 10 days storage at 4 deg C is from about 5.8 to about 5.9. It is surprisingly found to be effective at relatively low levels. Typically, carboxymethylcelluose sodium is present in from about 10 to about 60 mg, preferably about 20 to about 50 mg, more preferably about 20 to about 40 mg, yet more preferably about 25 to about 35 mg, per unit amount of formulation. This is advantageous as this does not have an adverse effect on ease of reconstitution or viscosity-related properties such as pourability. Many hydrophilic gums do not disperse quickly because each particle becomes surrounded by a "gummy" layer that hinders further hydration. Carboxymethylcelluose sodium, in contrast, disperses readily at the levels used. Furthermore, its effect on pH is not significantly influenced by the level of its inclusion, varying only over 0.5 pH units, over a concentration range 10 to 50 mg/ per unit amount of formulation.

Various grades of carboxymethylcellulose sodium suitable for use in formulations of the present invention are avaiiabie commercially from a number of different suppliers. These may vary according to particle size, degree of polymerisation, degree of substitution and purity. Pharmaceutically acceptable grades are preferred. In addition, the pH of a 1% w/v solution thereof may vary between 6.5 and 8.5, according to the USP monograph (US Pharmacopeia 25, 2002). Preferred grades of carboxymethylcellulose sodium for use in formulations of the present invention are those which have a pH of between about 7.0 and about 7.5 (for a 1.0 % w/v solution). Suitable grades of carboxymethylcellulose sodium are available from suppliers such as Hercules Incorporated (Wilmington, Delaware, USA) for instance the products Aqualon® CMC and Blanose® CMC; and Akzo Nobel Functional Chemicals (3800 AE Amersfoort, The Netherlands).

An embodiment comprises about 600/43 mg amoxicillin/clavulanate and from about 25 to 35 mg, typically about 30 mg, carboxymethylcelluose sodium per unit amount of formulation. In a representative example, this corresponds to about 2.5 to about 3.5% by weight of the formulation, typically about 3%.

In a further embodiment, the formulation further comprises xanthan gum, to complement carboxymethylcellulose sodium. Xanthan gum has a more immediate onset of action. Preferably, xanthan gum is present in from about 1 to about 10mg, preferably from about 1 to about 5 mg, more preferably from about 1 to about 4 mg, typically from about 2.5 to about 3.5 mg per unit amount of formulation. A typical ratio of carboxymethylcelluose sodium to xanthan gum is from about 12:1 to about 8:1, more typically about 10:1 by weight; for example about 30 mg carboxymethylcellulose sodium and about 3 mg xanthan gum, per unit amount of formulation.

Further excipients include a diluent (for clavulanate), a desiccant, a glidant, a flavouring agent and a sweetener.

Typically, silicon dioxide is included as a diluent and a desiccant, present in total from about 140 to about 190 mg (taking into account any contribution from silicon dioxide due to using a blend of potassium clavulanate and silicon dioxide, for instance a 1:1 blend) per unit amount of formulation.

Typically, the glidant is colloidal silicon dioxide, present in from about 30 to about 50 mg, preferably about 30 to about 40 mg, typically about 35 mg per unit amount of formulation, for formulations with up to 900 mg of amoxicillin. For formulations with a higher level of amoxicillin, it is found useful to incorporate higher levels, to maintain flowability of the product, for instance from about 60 to about 80mg, preferably from about 65 to about 80mg per unit amount of formulation.

Typically, the sweetener is an artificial sweetener such as aspartame, present in the range from about 10 to about 15 mg, typically about 12.5 mg per unit amount of formulation.

Typically, the flavouring agent is a fruit flavour, for instance the creamy strawberry flavour described in WO 01/13883.

A further embodiment comprises amoxicillin present in from about 700 to about 1250 mg, for instance about 800, 1000 or 1200 mg, potassium clavulanate present in from about 40 to 90 mg, for instance about 800/50, 800/57, 1000/62.5, and1200/43 mg; carboxymethylcellulose sodium present in from about 20 to about 50 mg, preferably about 20 to about 40 mg, more preferably about 25 to about 35 mg, typically about 30 mg; and xanthan gum present in from about 1 to about 10 mg, preferably from about 1 to about 5 mg, more preferably from about 1 to about 4 mg, typically about 3 mg; more preferably about 30 mg carboxymethylcellulose sodium and about 3 mg xanthan gum, per unit amount of formulation.

A further embodiment comprises amoxicillin present in about 600 mg; potassium clavulanate present in about 43 mg; carboxymethylcellulose sodium present in from about 20 to about 40 mg, preferably about 25 to about 35 mg, typically about 30 mg; and xanthan gum present in from about 1 to about 10 mg, preferably about 1 to about 5 mg, more preferably about 1 to about 4 mg, typically about 3 mg per unit amount of formulation; more preferably about 30 mg carboxymethylcellulose sodium and about 3 mg xanthan gum, per unit amount of formulation.

In further embodiments of the present invention, the excipients consist essentially of silicon dioxide, carboxymethylcellulose sodium, xanthan gum, colloidal silicon dioxide, an artificial sweetener and a flavouring agent, as hereinbefore defined.

As used herein, the term "unit amount of formulation " refers to the amount of formulation in the dry state which is reconstituted with water to provide 5 ml of suspension.

In the formulations of the invention, amoxicillin is in the form amoxicillin trihydrate. Preferably, the amoxicillin trihydrate is used as a pelletised form, for instance the form described in WO 02/49618 (SmithKline Beecham). Preferably, the equilibrium relative humidity (ERH) of the amoxicillin trihydrate used as raw material is carefully controlled by appropriate drying so that it does not adversely affect other aspects of the formulation. Preferably the ERH is less than less than 30%, most preferably from 10 to 20%.

Clavulanate is preferably in the form of potassium clavulanate. Potassium clavulanate is generally supplied in admixture with silicon dioxide as diluent, typically as a 1:1 blend. Potassium clavulanate is extremely moisture-sensitive and should be stored and handled in conditions of 30% RH or less, ideally as low as possible.

The weight ratios of amoxicillin:clavulanate expressed herein are as free acid equivalent.

Generally, the active materials amoxicillin trihydrate and potassium clavulanate are present in from about 70 to about 85% by weight of the initial formulation (using the absolute weights of amoxicillin trihydrate and potassium clavulanate).

Preferably, excipients are present in from about 15 to about 30% by weight of the initial formulation.

Formulations of the present invention may be manufactured using techniques which are generally conventional in the manufacture of dry powder formulations for reconstitution into aqueous suspensions. Typically, the dry, powdered ingredients are mixed together in a single step, in a blender, to give a homogeneous blend. This blend is then filled into suitable containers, such that each has a defined weight of product. In another aspect, the process may involve a preliminary step of forming granules from a subset of ingredients, for instance some or all of the amoxicillin content and the clavulanate.

Formulations of the present invention are preferably provided in an atmospheric moisture-proof container, for instance a glass or plastic bottle, for reconstitution with water or other suitable aqueous medium shortly prior to use. Preferably, a dry powder formulation is provided in a bottle with a moisture-proof cap.

Formulations of the present invention are primarily intended for administration to paediatric patients and will therefore preferably be accordingly adapted.

For convenience of dosing, the amount of powder provided in a bottle and the volume of water used to reconstitute the powder are determined so that a unit dosage is provided in a convenient volume, for instance from 2.5 to 10ml, typically about 5ml of reconstituted suspension. Typically, the bottle will comprise the whole course of therapy, so that reconstituted suspension will be a multiple dose suspension. Such administration may be by a spoon, for instance a calibrated spoon or a graduated measuring cup, a blunt-ended syringe or a calibrated dosing pump.

It will be appreciated that when specific amounts of clavulanate have been mentioned hereinbefore, such as 43 mg, 50 mg, 57 mg, 62.5 mg etc, these reflect a nominal amount and that, in practice, it may be necessary to include a overage of up to 10%, preferably up to 8%, more preferably up to 5%, based on the nominal weight of clavulanate, to allow for a controlled and acceptable degree of degradation. The present invention includes all such overage.

It will be further appreciated that in practice, to accommodate manufacturing practice, it may be necessary to allow for a fill weight overage, so that the amounts hereinbefore defined per unit amount of formulation may vary by up to 10%, typically a consistent variation across all ingredients of the formulation. The present invention includes all such variation.

It will be appreciated by the skilled person that when ratios of amoxicillin to clavulanate are mentioned, for instance 14:1, the ratio is a nominal, target ratio, and that, in practice, the ratio in the formulation may vary from this nominal value, for instance by up to about ±10%, preferably up to ±8%, in the number for amoxicillin, to accommodate the need to provide an overage to allow for degradation and also to reflect any subsequent degradation, in one or both actives. Thus, for instance, a ratio of weights of amoxicillin and clavulanate in a formulation which falls within the range 13:1 to 15:1 corresponds to a nominal ratio of 14:1. The present invention includes all such variation.

Formulations of this invention may be provided for the treatment of bacterial infections generally in children, for example one or more of *inter alia* upper respiratory tract infections, lower respiratory tract infections, genito-urinary tract infections and skin and soft tissue infections. Formulations of the present invention are suited to the treatment of upper respiratory tract infections in children, for instance otitis media and sinusitis, in particular acute otitis media, which infections are often caused by *S pneumonia,* including penicillin non-susceptible *S pneumonia* and penicillin resisitant *S pneumonia, H influenzae* and *M catarrhalis*. It will be appreciated that it is usual to treat such patients with body weight adjusted dosages. Thus, by way of example, a dosage regimen of 90/6.4 (ratio 14:1) mg/kg/day, in divided doses every 12 h (BID) may be provided by a suspension of the present invention comprising 600/43 mg/5 ml or 800/57 mg/5ml, if a lesser volume of unit dose is desired. A dosage regimen of 135/6.75 (ratio 20:1) mg/kg/day, in divided doses every 12h (BID) may be provided by a suspension of the present invention comprising 800/40 mg/5 ml or 1000/50 mg/5 ml. A dosage regimen of 150/10 (ratio 15:1) mg/kg/day, in divided doses every 8 or 12h (TID or BID) may be provided by a suspension of the present invention comprising 750/50 mg/5 ml. A dosage regimen of 160/10 (ratio 16:1) mg/kg/day, in divided doses every 8 or 12h (TID or BID) may be provided by a suspension of the present invention comprising 800/50 mg/5 ml or 1000/62.5 mg/5ml. Suitably, the multiple dose suspension is provided in sufficient amount to cover the course of therapy, typically from 7 to 10 days.

As used herein, the terms "paediatric" and "children" cover the age range from newly born to adolescent, for instance up to about 40 kg bodyweight, when it would be more appropriate to use an adult, for instance a tablet, formulation.

The invention will now be described by way of example.

### Example 1A - 600/43 mg/5ml suspension formulation

| **Ingredient** | **(mg/5 ml)** |
|---|---|
| *Amoxicillin Trihydrate (corresponding to 600 mg free acid) | 697.67 |
| *Clavulanate Potassium/Silicon Dioxide 1:1 Blend (corresponding to 42.9 mg free acid) | 113.00 (includes 8% overage) |
| Xanthan Gum, NF | 3.00 |
| Aspartame, NF | 12.50 |
| **Silicon Dioxide, NF | 92.82 |
| Colloidal Silicon Dioxide, NF | 35.00 |
| Sodium Carboxymethylcellulose, NF | 30.00 |
| Strawberry Flavor | 26.00 |
| *Total Dry Powder Fill Weight per unit amount* | *1010* |

| | |
|---|---|
| * Based on amoxicillin trihydrate at 86% amoxicillin (free acid) content and clavulanate potassium/silicon dioxide 1:1 blend at 41% clavulanic acid content. | |
| ** The quantity of silicon dioxide is adjusted per the amoxicillin trihydrate and clavulanate potassium/silicon dioxide blend active contents in order to maintain a constant dose weight. | |

In the above example, it will be appreciated that the amount of sodium carboxymethyl cellulose per unit amount of formulation is 30.00 mg.

It will be appreciated that in practice, a fill weight overage per unit amount may be included, so that a total dry powder fill weight per unit amount may increase by up to 10%, for instance to a total dry powder fill weight per unit amount of about 2000 mg

The suspension of Example 1A has an intial pH of about 5.2, compared with the product Augmentin ES 600 which has an intial pH of about 4.7. Furthermore, clavulanate declines by about 10% over a ten day period of storage at 4 deg C, compared with a loss of about 15% for the product Augmentin ES 600.

### Example 1B - 800/57 mg/5ml suspension formulation

| **Ingredient** | **(mg/5 ml)** |
|---|---|
| *Amoxicillin Trihydrate (corresponding to 600 mg free acid) | 930.23 |
| *Clavulanate Potassium/Silicon Dioxide 1:1 Blend (corresponding to 42.9 mg free acid) | 150.15 (includes 8% overage) |
| Xanthan Gum, NF | 3.00 |
| Aspartame, NF | 12.50 |
| **Silicon Dioxide, NF | 98.12 |
| Colloidal Silicon Dioxide, NF | 50.00 |
| Sodium Carboxymethylcellulose, NF | 30.00 |
| Strawberry Flavor | 26.00 |
| *Total Dry Powder Fill Weight per unit amount* | *1300* |

| | |
|---|---|
| * Based on amoxicillin trihydrate at 86% amoxicillin (free acid) content and clavulanate potassium/silicon dioxide 1:1 blend at 41% clavulanic acid content. | |
| ** The quantity of silicon dioxide is adjusted per the amoxicillin trihydrate and clavulanate potassium/silicon dioxide blend active contents in order to maintain a constant dose weight. | |

In the above example, it will be appreciated that the amount of sodium carboxymethyl cellulose per unit amount of formulation is 30.00 mg.

It will be also be appreciated that the above formulations of Examples 1A and 1B comprise amoxicillin trihydrate and potassium clavulanate in a ratio which is equivalent to a ratio of 14:1 (based on the weights of the corresponding free acids).

### Examples 2 to 4 - Formulations comprising 800/43, 1000/43 and 1200/43 mg/5ml amoxicillin and clavulanate

| **Ingredient** | **mg/5mL for '800' Product** | **mg/5mL for '1000' Product** | **mg/5mL for '1200' Product** |
|---|---|---|---|
| Amoxicillin Trihydrate | 930.23 | 1162.79 | 1395.35 |
| (corresponding to free acid) | (800) | (1000) | (1200) |
| Kclav./Syloid Blend | 113.00 | 113.00 | 113.00 |
| (corresponding to free acid) | (43) | (43) | (43) |
| Xanthan Gum | 3.00 | 3.00 | 3.00 |
| Aspartame | 12.50 | 12.50 | 12.50 |
| Micronized Silica Gel, 9 µ | 100.27 | 107.71 | 105.15 |
| Colloidal Silicon Dioxide | 35.00 | 35.00 | 35.00 |
| Sodium CMC | 30.00 | 30.00 | 30.00 |
| Strawberry Flavor | 26.00 | 26.00 | 26.00 |
| **Final Dose Wt.** (unit amount of formulation) | **1250 mg** | **1490 mg** | **1720 mg** |
| **Initial pH** | **5.20** | **5.11** | **5.05** |
| **10 day pH** | **5.83** | **5.82** | **5.82** |
| Based on an Amoxicillin potency of 86.0% pfa. and Clavulanate potency of 41.0%. | | | |

### Example 5

The impact of adding carboxymethylcellulose sodium to a series of typical formulations comprising amoxicillin and clavulanate on the loss of clavulanate in a reconstituted suspension over a period of 10 days was examined. The prototype formulations evaluated corresponded to those of Examples 1A and 2 to 4, with amoxicillin from 600 to 1200 mg/5ml and a constant level of clavulanate of 43 mg/5ml. In addition, a furtherformulation, with similar amounts of excipients and comprising 400 mg/5 ml, and a reference formulation comprising 200 mg/5ml, were also included, for comparison.

Formulations were constituted with water to the defined concentrations. Samples were taken at the beginning and after 10 days storage (at 4°C).

Samples were analysed for clavulanate concentration using HPLC and UV detection. In addition, the pH of the suspension was measured at the same time points.

The results are presented in the Table below for both pH change and clavulanate loss over 10 days.

**Table**

| **Formulation** | **0 day** | **pH 10 day** | **Diff** | **Clavulanate 10 Day Loss (%)** | **Reduction / (%)** |
|---|---|---|---|---|---|
| '200' | 4.60 | 6.13 | 1.53 | 9.4 | |
| '200' w/CMC | 5.48 | 5.91 | 0.43 | 8.6 | 0.8 / 8.5 |
| '400' | 4.46 | 6.05 | 1.59 | 12.0 | |
| '400' w/CMC | 5.40 | 5.87 | 0.47 | 9.1 | 2.9 / 24.2 |
| '600' | 4.35 | 6.00 | 1.65 | 11.8 | |
| '600' w/CMC | 5.30 | 5.84 | 0.54 | 9.5 | 2.3 / 19.5 |
| '800' ' | 4.25 | 5.90 | 1.65 | 13.7 | |
| 800' w/CMC | 5.20 | 5.83 | 0.63 | 10.2 | 3.5 / 25.5 |
| '1000' | 4.17 | 5.86 | 1.69 | 15.0 | |
| '1000' w/CMC | 5.11 | 5.82 | 0.71 | 9.6 | 5.4 / 36 |
| '1200' | 4.11 | 5.82 | 1.71 | 16.2 | |
| '1200' w/CMC | 5.05 | 5.82 | 0.77 | 11.4 | 4.8 / 29.6 |
| w/CMC - formulation with carboxymethylcellulose sodium | | | | | |

The results above show that over a range of concentrations of amoxicillin from 200 to 1200 mg/ 5ml the addition of carboxymethylcellulose sodium reduced the % loss of clavulanate. Whilst the improvement in the loss of clavulanate over 10 days by adding carboxymethylcellulose sodium is only slight for the formulation having 200mg/5ml amoxicillin, it is increasingly substantial for formulations with higher concentrations, in particular for concentrations of 600, 800, 1000 and 1200 mg/5 ml.

Furthermore it was found that for formulations with carboxymethylcellulose sodium, the pH starts out in the region of optimum stability (pH 5.0 to 5.5), falling to a final pH of 5.8 to 5.9. In comparison, corresponding formulations without carboxymethylcellulose sodium had an initial pH of 4.1 to 4.6 and a final pH of 5.8 to 6.1.

## Claims

1. A pharmaceutical formulation of amoxicillin and clavulanate provided as a dry powder composition adapted for reconstitution with water into a multiple dose suspension which comprises from about 400 to about 1250 mg amoxicillin and from about 40 to about 90 mg clavulanate per unit amount of formulation, such that the ratio of amoxicillin to clavulanate is at least 10:1, and which further comprises a pH stabilising agent which is carboxymethylcelluose sodium and pharmaceutically acceptable excipients.

2. A pharmaceutical formulation as claimed in claim 1 in which the suspension comprises from about 500 to about 1250 mg amoxicillin and from about 40 to about 90 mg clavulanate per unit amount of formulation, such that the ratio of amoxicillin to clavulanate is at least 12:1.

3. A pharmaceutical formulation as claimed in claim 1 or 2 in which the suspension comprises from about 550 to about 1250 mg amoxicillin and from about 40 to about 90 mg clavulanate per unit amount of formulation, such that the ratio of amoxicillin to clavulanate is at least 14:1.

4. A pharmaceutical formulation as claimed in any one of claims 1 to 3 in which the suspension comprises from about 700 to about 1250 mg amoxicillin and from about 40 to about 90 mg clavulanate per unit amount of formulation, such that the ratio of amoxicillin to clavulanate is at least 14:1.

5. A formulation as claimed in any one of claims 1 to 4 in which clavulanate is in the range about 40 to about 60 mg per unit amount of formulation.

6. A formulation as claimed in any one of claims 1 to 5 in which the ratio of amoxicillin to clavulanate is in the range about 12:1 to about 30:1.

7. A formulation as claimed in any one of claims 1 to 6 in which ratio of amoxicillin and clavulanate is about 14:1 or about 16:1.

8. A formulation as claimed in in any one of claims 1 to 7 in which carboxymethylcelluose sodium is present in from about 20 to about 60mg per unit amount of formulation.

9. A formulation as claimed in any one of claims 1 to 8 in which carboxymethylcelluose sodium is present in from about 20 to about 50mg per unit amount of formulation.

10. A formulation as claimed in in any one of claims 1 to 9 in which carboxymethylcelluose sodium is present in from about 25 to about 35 mg per unit amount of formulation.

11. A formulation as claimed in any one of claims 1 to 10 which further comprises xanthan gum present in from about 1 to about 10mg, preferably about 1 to about 5mg, more preferably about 1 to about 4 mg, typically about 3mg per unit amount of formulation.

12. A formulation as claimed in in any one of claims 1 to 3 which comprises amoxicillin present in from about 550 to about 1250 mg, potassium clavulanate present in from about 40 to about 90 mg, carboxymethylcellulose sodium present in from about 20 to about 50mg, and xanthan gum present in from about 1 to about 10mg per unit amount of formulation.

13. A formulation as claimed in in any one of claims 1 to 12 which comprises amoxicillin (corresponding to about 600 mg), potassium clavulanate (corresponding to about 43 mg), carboxymethylcellulose sodium (present in from about 20 to about 40mg) and xanthan gum (present in from about 1 to about 10mg) per unit amount of formulation.

14. A pharmaceutical formulation as claimed in any one of claims 1 to 13 and in which the excipients consist essentially of silicon dioxide, xanthan gum, colloidal silicon dioxide, an artificial sweetener and a flavouring agent.

15. A pharmaceutical formulation of amoxicillin and clavulanate provided as a reconstituted suspension which has a concentration in the range about 400 to about1250 mg amoxiciliin and about 40 to about 90 mg clavulanate per 5 mi of reconstituted suspension, such that the ratio of amoxicillin to clavulanate is at least 10:1, and which further comprises a pH stabilising agent which is carboxymethylcelluose sodium.

16. The use of carboxymethylcellulose sodium in reducing the amount of degradation of clavulanate in a reconstituted suspension comprising from about 400 to 1250 mg amoxicillin and from about 40 to about 90 mg clavulanate per 5 ml of reconstituted suspension, such that the ratio of amoxicillin to clavulanate is at least 10:1.

17. The use of carboxymethylcellulose sodium in stabilising the pH of a reconstituted suspension comprising from about 400 to about 1250 mg amoxicillin and from about 40 to about 90mg clavulanate per 5 ml of reconstituted suspension, such that the ratio of amoxicillin to clavulanate is at least 10:1, thereby reducing the amount of degradation of clavulanate.

18. A formulation as claimed in any one of claims 1 to 15 for treating bacterial infections.

19. A method of treating bacterial infection in paediatric patients which method comprises administering a therapeutically effective amount of a formulation as defined in any one of claims 1 to 15 to a patient in need thereof.
